# EUROPEAN PATENT SPECIFICATION

(11) **EP 1 733 697 B1**
(45) Date of publication and mention of the grant of the patent: **29.08.2018**
(21) Application number: 05012902.2
(22) Date of filing: 15.06.2005
(51) Int. Cl.: A61C 5/60

(54) **Applicator for a fluid dental composition**
Applikator für eine flüssige, dentale Zusammensetzung
Applicateur pour une composition dentaire liquide

(43) Date of publication of application: 20.12.2006
(73) Proprietor: DENTSPLY DETREY GmbH, 78467 Konstanz (DE)
(72) Inventor: Evers, Markus, 78476 Allensbach (DE); Iwatschenko, Peter, 90542 Eckental (DE)
(74) Representative: Hartz, Nikolai

(56) References cited:
- EP-A- 1 526 092
- US-B2- 6 685 013

## Description

### Field of the invention

The present invention relates to an applicator for a unit dose of a fluid dental composition. The present invention also relates to a process for preparing an applicator of the invention. Moreover, the present invention relates to a system comprising a plurality of disposable applicators of the invention. The fluid dental compositions may be selected from a dental adhesive, a dental primer, a dental sealant, a dental echant, a dental varnish, a composite, a staining solution for hard tissue having caries, or a local anaestetic composition. An applicator having the features according to the preamble of claim 1 is known from EP-A 1 526 092.

### Background of the invention

In the dental field dental compositions having fluidity are provided in bottles, sealed storage containers as disclosed in EP 1 153 579 A2, US 6,685,013 B2 and US 2003/0,146,117 A1, an applicator device as disclosed in WO 03/061508, or cartridges as disclosed in US 6,503,084 B2 or US 2003/0198918 A1.

A direct application of dental composition to an application site, e.g. a tooth or a restoration, is not possible from bottles or the storage containers disclosed in EP 1 153 579 A2, US 6,685,013 B2 or US 2003/0146117 A1. Therefore, an additional application device such as a brush is necessary for applying the dental composition to the application site. US 6,685,013 B2 discloses a dose package for applying a material with an applicator having a separate material and applicator portion. In an embodiment, a material well is squeezed to dispense the material onto an applicator. US 2003/0146117 Aldiscloses a device for storing and applying dental composition, which comprises a first and second film that define at least one first chamber that receives a first substance, and a pouch separated from the chamber for withdrawing the first substance. The device of US 2003/0146117 A1 does not have any rigidity.

The applicator device and cartridge disclosed in WO 03/061508 and US 6,503,084 B2, respectively, allow direct application of dental composition to an application site. However, the applicator device of WO 03/061508 is a device comprising a valve arid a rather complicated control and dispense equipment, whereby a large amount of dental composition is stored in a reservoir of the applicator device. The cartridges disclosed in US 6,503,084 B2 are designed for paste-like or honey-like dental compositions having high viscosity and only low fluidity, such as dental filling materials. Moreover, also a rather complicated application ejector device as disclosed in EP 0 744 161 A1 is necessary for pressing the dental composition out of the cartridge to an application site. Further, the use of the applicator device of WO 03/061508 and the cartridges of US 6,503,084 B2 do not allow the application of a thin layer of a dental composition having fluidity. Further, the known applicator device and system of cartridge and application ejector device do not provide the possibility to disperse a thin layer of applied material evenly on the surface of the application site. Particularly, the known applicator device and system of cartridge and application ejector do not provide the possibility to rub the dental composition into the application site. Moreover, the practical use of the known applicator device and the cartridge has shown that these containers are not solvent-tight. US 2003/0198918 discloses a dental composition container having a porous or foam flow through applicator connected at the discharge end thereof, and having a body portion containing a low viscosity dental composition. A plunger or piston is provided within the body portion to extrude the dental composition through the discharge orifice and attached flow through applicator. The container known from US 2003/0198918 requires a special tool for displacing the plunger or piston. US 2003/0198918 also discloses a sealed dental container or ampule that is squeezable.

Further, a device for storing and dispensing small quantities of liquids, e.g. for single-use, were disclosed in US 6,105 761 for a single-part material and in WO 01/64544 for a multi-part material. In this specification a single-part material means a material consisting of one or more than one component, whereby the material is stored in one compartment. A multi-part material means a material consisting of at least two components stored in at least two compartments. The single-use devices for storing and dispensing small amounts of liquids as disclosed in US 6,105,761 and WO 01/64544 are preferable in view of hygienic aspects when compared to multiple-use storing and application systems. Further, the single-use devices disclosed in US 6,105,761 and WO 01/64544 are solvent-tight. Furthermore, WO 01/64544 discloses that the device for storing and dispensing a fluid substance comprises an open ended pocket for dispensing the substance, whereby the pocket may act as an application device in case that the pocket is formed as a cannula. However, the device disclosed by WO 01/64544 is made of two sheets such that the pocket has only low rigidity even when formed as a cannula for direct application of the fluid substance. Thus, the pocket is not stable enough for rubbing the dental composition into the application site. Furthermore, WO 01/64544 discloses for repeated application of the fluid substance only the use of an additional application instrument such as a brush. Thus, application of the fluid substance in thin layers requires more steps by immersing the brush into the pocket. Namely, such brush is described only for immersing the brush into the fluid substance provided during use in the pocket. Furthermore, WO 01/64544 teaches to store the application instrument in the pocket during storage and during dispensing the fluid material into the pocket. Thus, WO 01/64544 does not disclose the provision of a storage and application device adapted for providing a flow resistance for the fluid substance such that the fluid substance is prevented from spurting out of the pocket during flow of the fluid material from its reservoir into the pocket. WO 01/64544 is silent to the provision of a suitable flow resistance for the fluid substance during its flow from the reservoir into the pocket. By contrast, it would be advantageously for the device of WO 01/64544 if the flow resistance for the fluid material would be decreased during its flow from the reservoir into the pocket, in order to sufficiently wet the brush stored in the pocket. However, in case that the open ended pocket formed as a cannula of the device disclosed in WO 01/64544 is employed without the provision of a flow-resistance for the dental composition, there arises the severe problem that the fluid substance will spurt out of the distal end of the pocket formed as a cannula. Thus, in the dental field direct application of the fluid substance may lead to unwanted spill of the fluid substance into the throat of a patient or onto sensitive tissue surrounding the application site.

GB-B 842,965 discloses an applicator for a liquid medicament which may be used to apply a small dose of a medicament to a rather small, defined and/or inaccessible region. The applicator comprises a flexible tube having a distal end with a discharge orifice covered by a cap. The tube contains a liquid medicament. In use, the proximal end of the tube is rendered open to atmospheric pressure so that the liquid may be discharged through the discharge orifice provided with a wick.

WO99/05987 discloses a hermetically sealed frangible container holding a tooth whitening solution. The frangible container is enclosed in a flexible vial with a closed end and an open end. The open end of the vial contains wicking applicator material.

WO01/62177 discloses an applicator assembly including an applicator having a tip and a cap initially extending over the tip and detachably connected to the applicator. The applicator includes a flexible portion that can be bent in an arc as the cap is detached.

A disposable applicator for a unit dose of a fluid dental composition, which comprises a fluid dental composition contained in a storage compartment is known from EP-A 1 526 092. Specifically, a device is disclosed in EP-A 1 526 092 for storing and dispensing a flowable substance, comprising a container comprising a base member and a cover member being sealingly connected with each other along the circumference of the container, at least one compartment for receiving said substance, and an open ended pocket area into which said substance is transferable from said at least one compartment.

It is the object of the present invention to provide a storage and application device which allows reliable storage of a dental composition having fluidity, even if a solvent is contained therein, and simple, reliable, fast, hygienic, and safe application of a small amount of the dental composition to an application site, even if the applicator is manipulated with only one hand.

### Summary of the invention

Accordingly, the present invention provides a disposable applicator for a unit dose of a fluid dental composition, which comprises a fluid dental composition contained in the storage compartment, characterized in that the applicator further comprises
(a) a rigid elongated member having a proximal end and a distal end, and
(b) a flexible film provided on a lateral surface of the rigid elongated member, said film defining walls of a storage compartment.

The present invention is based on the concept of providing a disposable applicator for a unit dose of a fluid dental composition which comprises a rigid elongated member and a flexible film which are combined so as to allow storage and discharge of the fluid dental composition. The rigid elongated member provides dimensional stability to the applicator, thereby improving the handling properties of the applicator. The rigid member comprises a system of channels for guiding the fluid dental composition during the introduction of the fluid dental composition into the applicator during manufacture, and for guiding the fluid dental composition during the application. Accordingly, the rigid elongated body comprises at least an application channel and a channel used for deaeration during charging of the dental composition into the applicator.

The applicator may comprise a single storage compartment. The applicator may also comprise two or more storage compartments whereby the flexible film provided on the lateral surface of the rigid elongated member defines walls of at least one of the storage compartments. Preferably, a flexible film defines walls of all storage compartments.

Preferably, the applicator comprises an additional channel used exclusively for filling fluid dental composition into the applicator. The channels provided by the rigid elongated body may be bores, or the channels may be open ducts which are complemented by further members of the applicators. Preferably, the rigid elongated member comprises a polymer, preferably selected from a polyamide, a polyolefin, a polycarbonate, and a polyester.

The flexible film is provided on a lateral surface, preferably an essentially planar lateral surface, of the rigid elongated member and provides curved side walls of a storage compartment for the fluid dental composition. The contours of the flexible film on the rigid elongated member define a storage portion wherein the storage compartment is provided. Upon compression of the storage compartment from the outside, pressure within the storage compartment is increased and a fluid communication between the storage compartment and a previously sealed channel in the rigid elongated member is established, and the fluid dental composition is extruded from the applicator. During storage, the fluid communication is prevented by a sealed portion. The sealed portion is preferably provided in the storage portion. Preferably, the seal is provided by the flexible film sealing a receiving orifice provided on the rigid elongated member. The storage compartment is provided at a portion of the rigid elongated body where the operator grips the applicator. Accordingly, by exerting pressure on the storage compartment from the outside, the grip of the operator of the applicator is improved and the control over the applicator is enhanced. The storage compartment has preferably an essentially tubular shape oriented along the longitudinal axis of the rigid elongated member. Preferably, the film provides three sides of the storage compartment, the fourth being provided by the rigid elongated member.

In a further embodiment, the flexible film may comprise more than one storage compartment. Accordingly, a corresponding number of bores for introducing dental compositions and for deaeration are provided at suitable locations of the storage portion. Preferably, a first storage compartment and a second storage compartment and corresponding bores are provided. The first storage compartment is preferably provided at a position which is more proximal than the second storage compartment. During storage, the first and second storage compartments are separated by the flexible film, optionally in cooperation with the rigid elongated member. When pressure is applied to the first storage compartment, a fluid flow communication is established between the first storage compartment and the second storage compartment. The fluid flow communication may be established by a selective detachment of the flexible film from the rigid elongated member so that an addition channel is established between the first storage compartment and the second storage compartment. Accordingly, the content of the first storage compartment is added to the second storage compartments. Moreover, the pressure applied to the storage compartments also provides a fluid flow communication between the storage compartment and a channel in the rigid elongated member whereby the combined fluid dental composition may be extruded from the applicator. During storage, the fluid communication is prevented by the a sealed portion. The sealed portion is preferably provided within the storage portion defined by the contour of the flexible film in the lateral surface of the elongated member. Preferably, the seal is provided by the flexible film sealing a receiving orifice provided on the rigid elongated member. This embodiment may be used for dental compositions containing reactive components which cannot be stored in the same storage compartment over an extended period of time. In the following, reference is made to the embodiment comprising a single storage compartment. However, the following disclosure equally applies to an embodiment having more than one storage compartment, in particular two storage compartments.

The flexible film provides a diffusion barrier for the dental composition to be received in a storage compartment defined at least partially by the flexible film. The film may be a composite foil comprising a polymeric and a metallic layer. The polymeric material comprises preferably a polyamide, a polyester, a polycyclic olefin such as Topas®, and/or a polyolefin. The metallic layer comprises preferably aluminum. Preferably, the composite foil has a deep-drawing property suitable for cold forming. More preferably, the composite foil is a laminate comprising as polymeric material a polycyclic olefin. Particularly preferred is an embodiment wherein a film is used which is a composite foil having a deep-drawing property and which is based on a polycyclic olefin layer, preferably of Topas®, an aluminum layer, and a polyolefin layer, preferably of polyethylene. A polyethylene terephthalate layer may be provided between the aluminum layer and the polyethylene layer.

In a preferred embodiment, the applicator further comprises a sealing member. The sealing member is provided on a lateral surface of the rigid elongated member preferably opposite from the storage compartment. The sealing member is preferably a rigid body having protrusions engaging with bores in the rigid elongated member. The sealing member may comprise one or more protrusions for engaging and sealing through bores extending from the storage compartment perpendicular to the longitudinal axis through the rigid elongated member.

The flexible film may be attached to the rigid elongated member by any suitable technique such as hot sealing or glueing. The attachment is preferably adjusted so that a selective detachment of the film is possible upon exerting pressure on the one or more storage compartments from the outside.

The selective detachment should provide a fluid communication between the one or more storage compartments and the receiving orifice whereby a fluid communication between the one or more storage compartments and a dispensing orifice at the tip portion of the elongated rigid member is established via the receiving orifice. A sealing portion which may be selectively detached upon compression of a storage compartment may be provided on the flexible film. In case of hot sealing, the sealing portion may be provided by using a lower temperature at the sealing portion as compared to the other portions which shall not be detached upon compression of the storage compartment. According to a further embodiment, a sealing portion may be provided by incorporating particles or chips of a peel-off film or other foreign particles or chips which reduce the adhesion of the film.

The receiving orifice is preferably provided on the rigid elongated member within the storage portion, but outside any storage compartment. The receiving orifice may be sealed by the flexible film during storage of the fluid dental composition. The seal of the receiving orifice may be broken by applying pressure to the one or more storage compartments defined by the flexible film, thereby providing a fluid communication between the storage compartment and the dispensing orifice through the bore.

In a preferred embodiment, the receiving orifice allows the fluid dental composition to enter into a first channel being essentially perpendicular to the longitudinal axis of the elongated member, and guiding the fluid dental composition to a second channel being essentially parallel to the longitudinal axis, and guiding the fluid dental composition to the dispensing orifice. In a further preferred embodiment, the second channel comprises a portion sealed by the sealing member.

The applicator according to the invention may further comprise a tip portion distal from the storage portion. The tip portion may be a cannula or needle integrally connected to the rigid elongated body. The tip portion may also be adapted for receiving a hollow injection needle e.g. for application of a local anaestetic stored in the applicator. The applicator comprises a channel providing fluid communication between a receiving orifice at the storage portion and a dispensing orifice at the tip portion. The channel may be defined by the rigid elongated body. However, the sealing member may also be used to define a wall of the channel providing openings of the storage compartment. The tip portion may also include a flexible portion that is located between the application tip and the storage portion. The flexible portion is deformable by finger pressure past its yield point to any one of a number of angular orientations, and once bent will substantially self-remain in a bent orientation without returning to its initially straight orientation. The flexible portion may be provided by one or more grooves that serve to facilitate bending of the shaft.

The applicator of the present invention provides a flow resistance for the dental composition such that the dental composition is prevented from spurting out of the distal end. This may be achieved, for example, by adapting the dimensions of the channel, particularly the length of the channel and/or the inner diameter of the channel, and/or the amount of dental composition contained in the single-use storage and application device. Thus, the velocity of dental composition flowing through the passage of the cannula may be adjusted. Particularly, the channel may have a length of 5 to 80 mm, preferably 10 to 60 mm. Furthermore, the channel may have an inner diameter of 0.1 to 1 mm, preferably 0.3 to 0.7 mm. Moreover, the tip portion may have an outer diameter of 0.5 to 2.5 mm, preferably 0.5 to 1.5 mm.

In a specific embodiment, the applicator may preferably comprise an application tip comprising fibers, bristles, a porous material, a wick, or a fabric in order to better control the application of the dental composition. According to this embodiment of the present invention the flow resistance for the dental composition such that the dental composition is prevented from spurting out of the application tip at the distal end is provided by adjusting the penetrability and capacity of the application tip. The application tip may comprise a sponge, bristles, a fleece, fibrous material, fibers, and/or filaments. Preferably, the bibulous material may be applied to the cannula according to the following process. A rigid elongated member, preferably of polymeric material, may be treated with plasma for activating the outer surface of the application tip. Then an adhesive may be applied to the activated surface. The activation may improve the adherence of the adhesive. Subsequently, air or an inert gas may be blown through the application tip and fibers, filaments, and/or chips, preferably of a polymeric material, more preferably of a polymer foam, are applied. This embodiment is particularly preferred, since penetrability and capacity of the material may be adjusted easily.

According to a further embodiment of the present invention, flow resistance for the dental composition such that said dental composition is prevented from spurting out of said distal end may be provided.

According to a further embodiment, the applicator comprises an injection needle which may be integrally connected to the rigid elongated member, or which may be attached to the applicator prior to use. The injection needle may be made of metal or a polymeric material.

Furthermore, the amount of dental composition for single-use is dependent on the purpose of the dental composition. Particularly, the dental composition may be an adhesive, a sealer, an etching gel, a varnish, a fluid composite, a local anaestetic, or a staining solution for tissue having caries.

The amount of the dental composition in the single-use storage and application device may range between 50 µl and 1.0 ml, preferably 75 µl and 750 µl, more preferably 100 µl and 500 µl. The applicator contains the dental composition, preferably in an amount sufficient for single use or application to one patient.

The applicator may further comprise a means for applying pressure to the storage compartment for extruding the fluid dental composition through the channel. The means is preferably provided outside the storage compartment. The means may be pivotally attached to the elongated body in a proximal position from the storage compartment so that the means may apply pressure to the storage compartment defined by the flexible film when pressure is exerted on the means. The means may be a protective cover for the flexible film defining the storage compartment.

The present invention also provides a process for preparing an applicator, which comprises
(a) providing a rigid elongated member having a proximal end and a distal end,
(b) providing a flexible film on a lateral surface of the rigid elongated member for defining walls of a storage compartment,
(c) introducing a fluid dental composition in the storage compartment through a through bore, and
(d) sealing the through bore with a sealing member for providing an applicator.

In a preferred embodiment, the fluid dental composition is introduced into the storage compartment through bores extending from the storage compartment perpendicular to the longitudinal axis of the elongated member. Preferably, at least two bores are provided in order for air entrapped in the storage compartment to be released through one bore when the dental composition is introduced into the storage compartment through another bore.

The present invention also provides a system of applicators comprising a plurality of disposable applicators for a unit dose of a fluid dental composition according to the present invention. Preferably, the fluid dental compositions are selected from a dental adhesive, a dental primer, a dental sealant, a dental echant, a dental varnish, a composite, a staining solution for hard tissue having caries, or a local anaestetic composition.

The present invention is also directed toward a method of applying a dental composition to tooth structure. The method includes the step of providing an applicator according to the present invention. The method also includes the steps of exerting pressure on the storage compartment for providing a fluid communication to the tip portion and contacting the tip portion with tooth structure and/or soft tissue in order to transfer at least a portion of the composition to the tooth structure and or soft tissue.

Preferably, the applicator is disposed of after a single use in order to avoid problems with cross-contamination. The applicator may also be used advantageously to dispense and apply a composition that cannot be feasiblely dispensed from a bulk container over a period of time, such as a composition that rapidly cures upon exposure to the atmosphere or to ambient light.

### Brief description of the drawings

Figures 1a-f show a first embodiment of an applicator according to the present invention.
Figure 2 shows an exploded view of a second embodiment of an applicator according to the present invention.
Figures 3a-f show different views of the second embodiment of an applicator according to the present invention.

### Detailed Description of the Preferred Embodiments

The present invention is now described in further detail with reference to the accompanying drawings. Corresponding or equivalent elements of the invention illustrated in different drawings are designated with the same reference numerals.

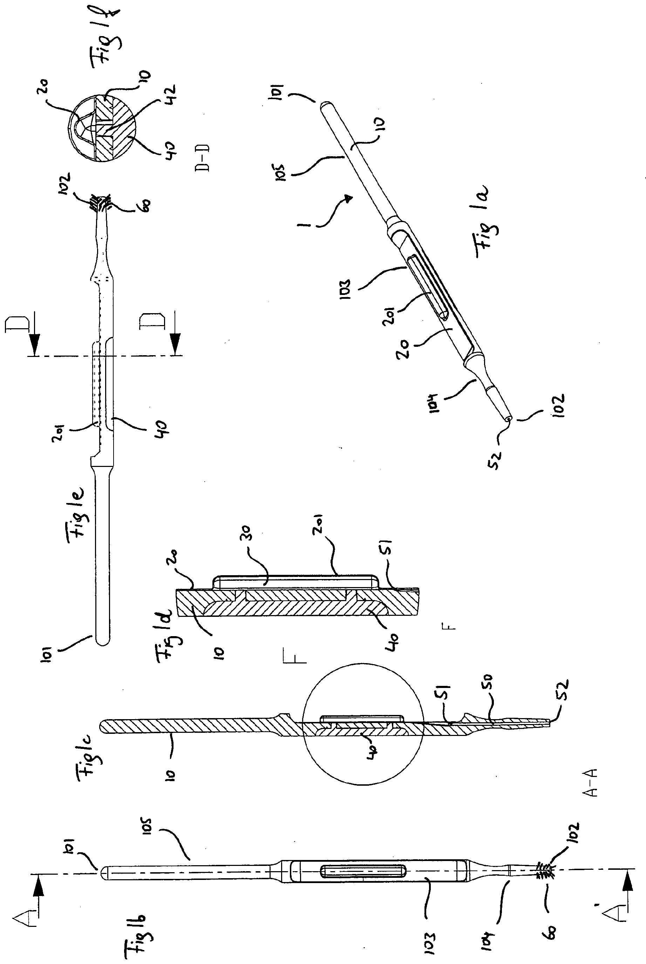

Fig. 1 shows a first embodiment of the disposable applicator according to the invention which is characterized by a receiving orifice introducing the fluid dental composition in a channel extending essentially parallel to the longitudinal axis A-A' of the applicator.

An applicator according to the invention is designated by reference numeral 1. As shown in Fig. 1a, the applicator 1 comprises a rigid elongated member 10 having a proximal end 101 and a distal end 102. The applicator further comprises a flexible film 20 provided on a lateral surface of the rigid elongated member 10. The flexible film defines by its contours a storage portion on the lateral surface of the elongated member 10. The flexible film 20 also defines walls of a storage compartment 201. A fluid dental composition 30 is contained in the storage compartment 201. The storage portion 103 separates a handle portion 105 proximal to the storage portion and a tip portion 104 distal from the storage portion 103. The storage portion 103 may serve at the same time as grip portion for holding the applicator 1 by the operator.

Figure 1b shows a top view of the applicator 1 according to the first embodiment of the invention. The applicator 1 has a longitudinal axis A-A' extending from the proximal end 101 to the distal end 102. The handle portion 105 and the tip portion 104 are essentially cylindrical or conical whereas the storage portion has a lateral surface which is essentially planar. The application tip 60 includes a material facilitating application of the fluid dental composition across the surface to which the composition is to be applied. There are no specific limitations regarding the material as long as it is compatible with the dental composition and functions to distribute the dental composition over the receiving surface. Suitable materials are small bristles or fibers that serve as a brush and that are applied to all or only part of the application tip 60. Fibers may be applied to the application tip 60 by a flocking process. The flocking can be carried out by any suitable technique. The fibers preferably allow uniform application of the composition over a surface regardless of whether the surface is irregular, rough or smooth, and apply the composition in the same way as a brush would. Alternatively, other types of material such as a porous material, a wick, or a fabric may be applied to the application tip 60 for facilitating spreading of the composition across a surface. Specific examples of such other materials include an open cell foam material such as polyurethane foam or synthetic sponge. Additional examples of suitable materials are woven and non-woven fabrics or gauzes. Micro-structured surfaces including micro-structured surfaces integrally formed as part of the application tip 60, may also be used.

As illustrated by Fig. 1c, the lateral planar surface is obtainable based on a recess extending essentially unto the plane of the longitudinal axis A-A'. According to the first embodiment, a receiving orifice 51 is provided on the distal end of the storage portion 103. The receiving orifice serves for introducing the fluid dental composition 30 into a channel 50 extending essentially parallel to longitudinal axis A-A' from the receiving orifice 51 to the dispensing orifice 52.

Fig 1d shows an enlarged cross-sectional side view of a section of the storage portion 103 including the storage compartment 201 containing a fluid dental composition 30. The flexible film 20 extends over the storage portion 103 and forms the storage compartment 201. The flexible film 20 is attached to the rigid elongated member 10 so that the fluid dental composition 30 may be stored without any diffusion of the dental composition from the storage compartment.

Fig. 1d indicates that the receiving orifice 51 is sealed by the flexible film 20 during storage and shipping of the fluid dental composition 30. Moreover, a sealing member 40 is provided on a lateral surface of the rigid elongated member 10 opposite from the storage compartment 201. The sealing member 40 is received in a recess portion opposite from the storage portion. The sealing member 40 has protrusions 41 and 42 which engage in bores extending through the rigid elongated member essentially perpendicular to the longitudinal axis A-A'.

As shown by Fig. 1e, the sealing member may be integrated into the outer contour of the rigid elongated member 10. The principal function of the sealing member 40 is the engaging and sealing of through bores in the rigid elongated body 10 after the storage compartment 201 was filled with the fluid dental composition 30. During storage, shipping and use of the applicator 1, the sealing member 40 serves as structural element enhancing the stability of the rigid elongated body. Accordingly, the sealing member 40 is preferably permanently attached to the rigid elongate body 10.

Fig. 1f is a cross-sectional view of the storage portion at a protrusion 42 of the sealing member which engages a through-bore of the rigid elongated member.

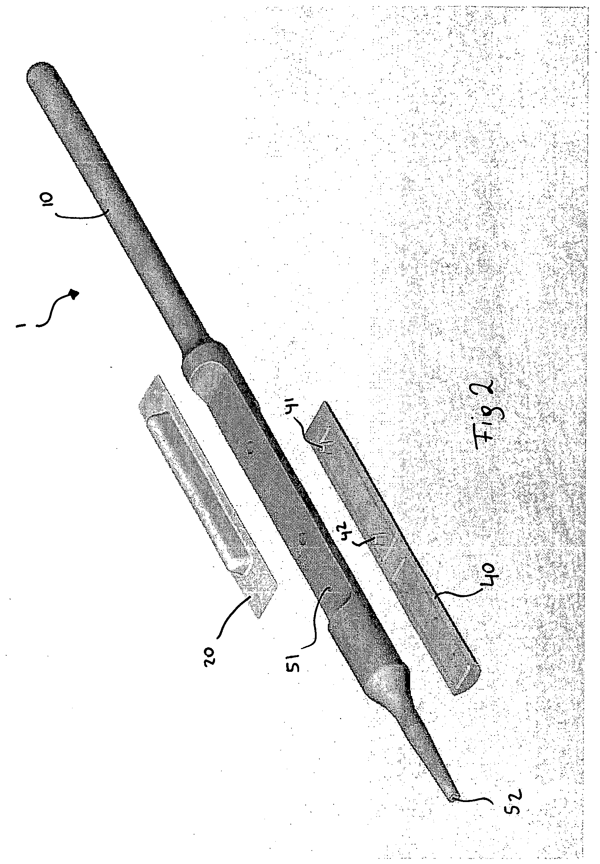

A second embodiment of the invention is illustrated in an exploded view in Fig. 2, wherein an applicator 1 comprises a rigid elongated body 10, a flexible film 20, and a sealing member 40. The second embodiment of the disposable applicator according to the invention is characterized by a receiving orifice introducing the fluid dental composition into a first channel portion (510) extending essentially perpendicular to the longitudinal axis A-A' of the applicator so that the receiving orifice 51 and the through bores used for filling the storage compartment are in the same plane. From the first channel portion, (510) the fluid dental composition is guided to a second channel portion (520) extending to the dispensing orifice.

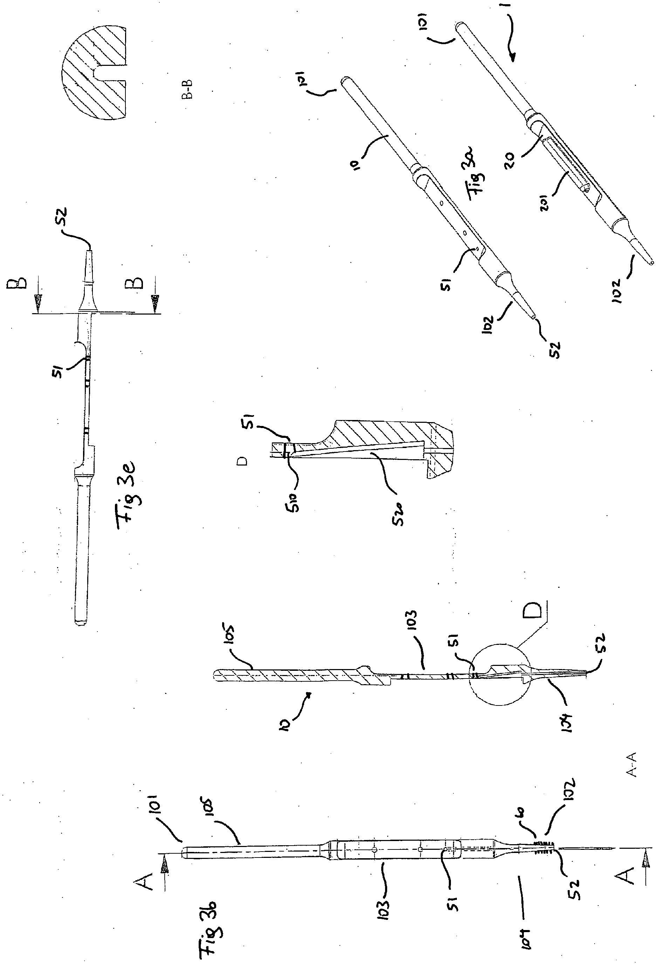

As shown in Fig. 3a, the applicator 1 comprises a rigid elongated member 10 having a proximal end 101 and a distal end 102.

Figure 3b shows a top view of the applicator 1 according to the second embodiment of the invention. The applicator 1 has a longitudinal axis A-A' extending from the proximal end 101 to the distal end 102. The handle portion 105 and the tip portion 104 are essentially cylindrical or conical whereas the storage portion 103 has a surface, which is essentially planar. The application tip 60 includes a material facilitating application of the fluid dental composition 30 across the surface to which the composition is to be applied. There are no specific limitations regarding the material as long as it is compatible with the dental composition and functions to distribute the dental composition over the receiving surface. The same material as used for the first embodiment of the invention may be used.

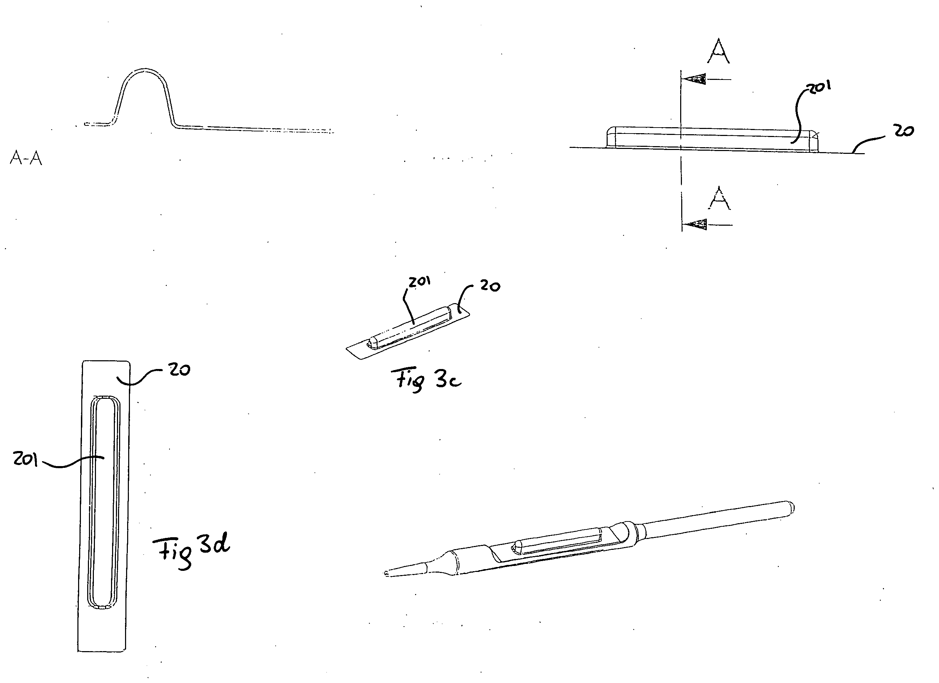

Figure 3c and figure 3d show a flexible film 20 to be attached on a lateral surface of the rigid elongated member 10. The flexible film 20 defines by its contours a corresponding storage portion 103 on the lateral surface of the elongated member 10. The flexible film 20 also defines walls of a storage compartment 201. A fluid dental composition 30 is contained in the storage compartment 201 when the flexible film 20 is attached to the rigid elongated body. The storage portion 103 separates a handle portion 105 proximal to the storage portion and a tip portion 104 distal from the storage portion 103. The storage portion 103 serves at the same time as grip portion for holding the applicator 1 by the operator.

As illustrated by Fig. 3e, the lateral surface is obtainable based on a recess extending essentially unto the plane of the longitudinal axis A-A' as in the case of the first embodiment. However, according to the second embodiment, a receiving orifice 51 is provided in the plane of the lateral surface on the distal end of the storage portion. The receiving orifice 51 serves for introducing the fluid dental composition 30 into a channel 50 extending essentially perpendicular to longitudinal axis A-A' from the receiving orifice 51 to the dispensing orifice 52.

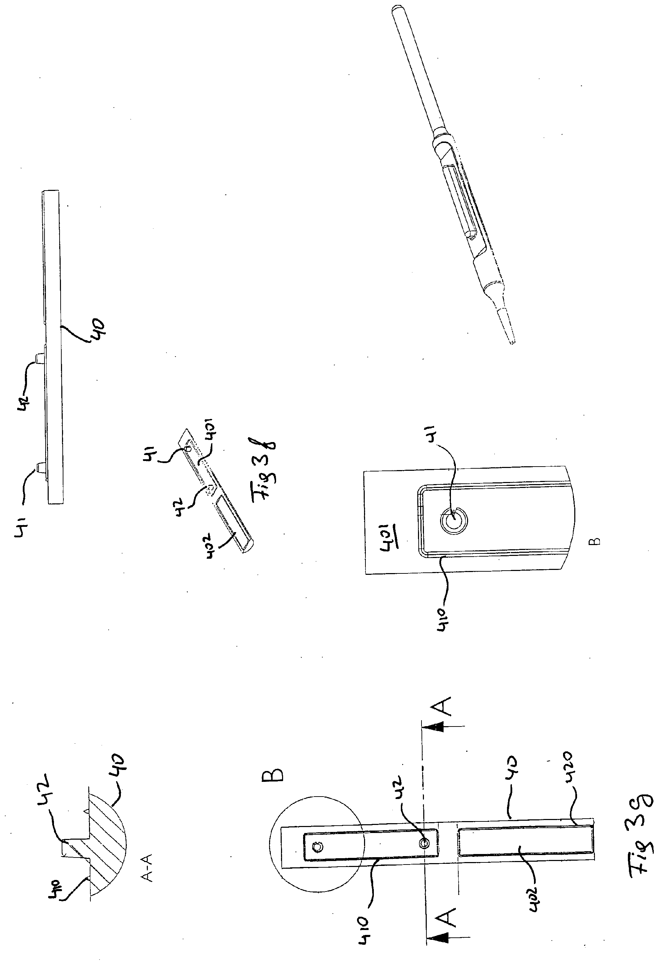

Figure 3f and figure 3g show a sealing member 40 to be fitted on a lateral surface of the rigid elongated member 10 opposite from the storage compartment 201. The sealing member 40 is received in a recess portion opposite from the storage portion 103. The sealing member 40 has two functional portions 401 and 402 according the second embodiment. Functional portion 401 corresponds to sealing member of the first embodiment and comprises the protrusions 41 and 42 which engage in bores extending through the rigid elongated member 10 essentially perpendicular to the longitudinal axis A-A'. The functional portion 402 provides a wall of a channel for guiding the fluid dental composition 30 from the receiving orifice 51 to the dispensing orifice 52. Accordingly, the principal function of the sealing member 40 is the engaging and sealing of through bores in the rigid elongated body after the storage compartment was filled with the fluid dental composition, and the guiding of the fluid dental composition 30 during use. The functional portions 401 and 402 may comprise sealing protrusions 410, 420 engaging with suitable sealing recesses of the rigid elongated body. The sealing member 40 may be integrated into the outer contour of the rigid elongated member 10. During storage, shipping and use of the applicator 1, the sealing member 40 serves as structural element enhancing the stability of the rigid elongated body 10. Accordingly, the sealing member 40 is preferably permanently attached to the rigid elongate body 10.

## Claims

1. A disposable applicator (1) for a unit dose of a fluid dental composition (30), which comprises a fluid dental composition (30) contained in a storage compartment (201), **characterized in that** the applicator (1) further comprises
(a) a rigid elongated member (10) having a proximal end (101) and a distal end (102),
(b) a flexible film (20) provided on a lateral surface of the rigid elongated member (10), said film defining walls of the storage compartment (201),.

2. The applicator according to claim 1, wherein the applicator further comprises (d) a sealing member (40) provided on a lateral surface of the rigid elongated member (10) opposite from the storage compartment (201).

3. The applicator according to any one of claims 1 or 2, wherein the elongated member (10) comprises a storage portion (103) defined by the flexible film (20) provided on the rigid member (10).

4. The applicator of claim 3, which further comprises a tip portion (104) distal from the storage portion (103).

5. The applicator of claim 4, which comprises a channel (50) providing fluid communication between a receiving orifice (51) at the storage portion (103) and a dispensing orifice (52) at the tip portion (104).

6. The applicator of claim 5, wherein the receiving orifice (51) is sealed by the flexible film (20) during storage of the fluid dental composition (30).

7. The applicator of claim 5 or 6, wherein the seal of the receiving orifice (51) may be broken by applying pressure to the storage compartment (201) defined by the flexible film (20), thereby providing a fluid communication between the storage compartment (201) and the dispensing orifice through (52) the channel (50).

8. The applicator of any one of claims 2 to 7, wherein the sealing member (40) comprises one or more protrusions (41, 42) for engaging and sealing through bores extending from the storage compartment (201) perpendicular to the longitudinal axis (A-A') through the rigid elongated member (10).

9. The applicator according to any one of the preceding claims wherein the applicator comprises an application tip (60) comprising fibers, bristles, a porous material, a wick, a fabric, a sponge, a fleece, and/or filaments.

10. The applicator according to any one of claims 5 to 8, wherein the tip portion comprises a hollow needle.

11. The applicator according to any one of the previous claims, which comprises a means for applying pressure from the outside on the storage compartment (201) for extruding the fluid dental composition (30) through the channel (50).

12. The applicator according to claim 11, wherein the means is pivotally attached to the elongated body in a proximal position from the storage compartment (201) so that the means may apply pressure to the storage compartment (201) defined by the flexible film (20) when pressure is exerted on the means.

13. The applicator according to claim 11, wherein the means is a protective cover for the flexible film defining the storage compartment.

14. The applicator according to any one of the preceding claims, wherein the receiving orifice (51) allows the fluid dental composition to enter into a first channel portion (510) being essentially perpendicular to the longitudinal axis of the elongated member (10), and guiding the fluid dental composition to a second channel portion (520) being essentially perpendicular to the first channel portion, and guiding the fluid dental composition to the dispensing orifice (52).

15. The applicator according to claim 14, wherein the second channel portion (520) comprises a portion (521) sealed by the sealing member.

16. The applicator according to any one of the preceding claims , which comprises at least two storage compartments.

17. A process for preparing an applicator according to any one of claims 1 to 16, which comprises
(a) providing a rigid elongated member (10) having a proximal end and a distal end,
(b) providing a flexible film (20) on a lateral surface of the rigid elongated member (10) for defining walls of a storage compartment (201),
(c) introducing a fluid dental composition (30) in the storage compartment (201) through a through bore, and
(d) sealing the through bore with a sealing member (40) for providing an applicator.

18. The process of claim 17, wherein the fluid dental composition is introduced into the storage compartment through bores extending from the storage compartment (201) perpendicular to the longitudinal axis (A-A') of the elongated member (10).

19. The process of claim 18, wherein at least two bores are provided in order for air entrapped in the storage compartment to be released through one bore when the dental composition is introduced into the storage compartment through another bore.

20. A system of applicators comprising a plurality of disposable applicators (1) for a unit dose of a fluid dental composition (30) as defined by any one of claims 1 to 16.

21. The system according to claim 20, wherein the fluid dental compositions are selected from a dental adhesive, a dental primer, a dental sealant, a dental echant, a dental varnish, a composite, a staining solution for hard tissue having caries, or a local anaestetic composition.

## Patentansprüche

1. Einwegapplikator (1) für eine Einheitsdosis einer flüssigen Dentalzusammensetzung (30), die eine flüssige Dentalzusammensetzung (30) umfasst, welche in einer Speicherkammer (201) enthalten ist, **dadurch gekennzeichnet, dass** der Applikator (1) ferner umfasst (a) ein starres längliches Element (10) mit einem proximalen Ende (101) und einem distalen Ende (102), (b) einen flexiblen Film (20), der auf einer Seitenfläche des starren länglichen Elements (10) vorgesehen ist, wobei der Film Wände der Speicherkammer (201) definiert.

2. Applikator nach Anspruch 1, wobei der Applikator ferner umfasst
(d) ein Dichtungselement (40), das auf einer Seitenfläche des starren länglichen Elements (10) gegenüber der Speicherkammer (201) vorgesehen ist.

3. Applikator nach einem der Ansprüche 1 oder 2, wobei das längliche Element (10) einen Speicherabschnitt (103) umfasst, der durch die auf dem starren Element (10) vorgesehene flexible Folie (20) definiert ist.

4. Applikator nach Anspruch 3, welcher ferner einen Endstückabschnitt (104) distal von dem Speicherabschnitt (103) umfasst.

5. Applikator nach Anspruch 4, welcher einen Kanal (50) umfasst, der eine Fluidverbindung zwischen einer Aufnahmeöffnung (51) am Speicherabschnitt (103) und einer Abgabeöffnung (52) am Endstückabschnitt (104) umfasst.

6. Applikator nach Anspruch 5, wobei die Aufnahmeöffnung (51) während des Speicherns der flüssigen Dentalzusammensetzung (30) durch die flexible Folie (20) abgedichtet ist.

7. Applikator nach Anspruch 5 oder 6, wobei die Dichtung der Aufnahmeöffnung (51) durch das Ausüben von Druck auf die Speicherkammer (201), die durch die flexible Folie (20) definiert ist, gebrochen werden kann und dadurch eine Fluidverbindung zwischen der Speicherkammer (201) und der Abgabeöffnung (52) durch den Kanal (50) bereitstellt.

8. Applikator nach einem der Ansprüche 2 bis 7, wobei das Dichtungselement (40) einen oder mehrere Vorsprünge (41, 42) zum Eingreifen in und Abdichten von Durchgangsbohrungen, die sich von der Speicherkammer (201) senkrecht zur Längsachse (A-A') durch das starre längliche Element (10) erstrecken, umfasst.

9. Applikator nach einem der vorstehenden Ansprüche, wobei der Applikator ein Anwendungsendstück (60) umfasst, das Fasern, Borsten, ein poröses Material, einen Docht, ein Gewebe, einen Schwamm, ein Vlies und/oder Fäden umfasst.

10. Applikator nach einem der Ansprüche 5 bis 8, wobei der Endstückabschnitt eine hohle Nadel umfasst.

11. Applikator nach einem der vorstehenden Ansprüche, welches ein Mittel zum Ausüben von Druck von außerhalb auf die Speicherkammer (201) zum Extrudieren der flüssigen Dentalzusammensetzung (30) durch den Kanal (50) umfasst.

12. Applikator nach Anspruch 11, wobei das Mittel an dem länglichen Körper in einer proximalen Position von der Speicherkammer (201) drehbar befestigt ist, sodass das Mittel Druck auf die Speicherkammer (201), die durch die flexible Folie (20) definiert ist, ausüben kann, wenn Druck auf das Mittel ausgeübt wird.

13. Applikator nach Anspruch 11, wobei das Mittel eine Schutzabdeckung für die flexible Folie ist, welche die Speicherkammer definiert.

14. Applikator nach einem der vorstehenden Ansprüche, wobei die Aufnahmeöffnung (51) ermöglicht, dass die flüssige Dentalzusammensetzung in einen ersten Kanalabschnitt (510) eintritt, der zur Längsachse des länglichen Elements (10) im Wesentlichen senkrecht ist, und die flüssige Dentalzusammensetzung zu einem zweiten Kanalabschnitt (520) lenkt, der zu dem ersten Kanalabschnitt im Wesentlichen senkrecht ist, und die flüssige Dentalzusammensetzung zur Abgabeöffnung (52) lenkt.

15. Applikator nach Anspruch 14, wobei der zweite Kanalabschnitt (520) einen Abschnitt (521) umfasst, der durch das Dichtungselement abgedichtet ist.

16. Applikator nach einem der vorstehenden Ansprüche, welcher mindestens zwei Speicherkammern umfasst.

17. Prozess zum Herstellen eines Applikators nach einem der Ansprüche 1 bis 16, der umfasst
(a) Bereitstellen eines starren länglichen Elements (10) mit einem proximalen Ende und einem distalen Ende,
(b) Bereitstellen einer flexiblen Folie (20) auf einer Seitenfläche des starren länglichen Elements (10) zum Definieren von Wänden einer Speicherkammer (201),
(c) Einführen einer flüssigen Dentalzusammensetzung (30) in die Speicherkammer (201) durch eine Durchgangsbohrung und
(d) Abdichten der Durchgangsbohrung mit einem Dichtungselement (40) zum Bereitstellen eines Applikators.

18. Prozess nach Anspruch 17, wobei die flüssige Dentalzusammensetzung in die Speicherkammer durch Bohrungen eingeführt wird, die sich von der Speicherkammer (201) senkrecht zur Längsachse (A-A') des länglichen Elements (10) erstrecken.

19. Prozess nach Anspruch 18, wobei mindestens zwei Bohrungen vorgesehen sind, um Luft, die in der Speicherkammer eingeschlossen ist, durch eine Bohrung freizugeben, wenn die Dentalzusammensetzung durch eine andere Bohrung in die Speicherkammer eingeführt wird.

20. System von Applikatoren, das mehrere Einwegapplikatoren (1) für eine Einheitsdosis einer flüssigen Dentalzusammensetzung (30) nach einem der Ansprüche 1 bis 16 umfasst.

21. System nach Anspruch 20, wobei die flüssigen Dentalzusammensetzungen aus einem Dentalklebstoff, einem Dentalprimer, einer Dentalversiegelung, einem Dentalätzmittel, einem Dentallack, einer Zusammensetzung, einer Färbelösung für Hartgewebe, das Karies aufweist, oder einer örtlichen Betäubungszusammensetzung ausgewählt sind.

## Revendications

1. Applicateur jetable (1) pour une dose unitaire de composition dentaire fluide (30), qui comprend une composition dentaire fluide (30) contenue dans un compartiment de stockage (201), **caractérisé en ce que** l'applicateur (1) comprend en outre
(a) un élément allongé rigide (10) ayant une extrémité proximale (101) et une extrémité distale (102),
(b) un film flexible (20) prévu sur une surface latérale de l'élément allongé rigide (10), ledit film définissant des parois du compartiment de stockage (201).

2. Applicateur selon la revendication 1, l'applicateur comprenant en outre
(d) un élément d'étanchéité (40) prévu sur une surface latérale de l'élément allongé rigide (10) opposée au compartiment de stockage (201).

3. Applicateur selon l'une quelconque des revendications 1 ou 2, dans lequel l'élément allongé (10) comprend une partie de stockage (103) définie par le film flexible (20) prévu sur l'élément rigide (10).

4. Applicateur de la revendication 3, qui comprend en outre une partie de pointe (104) distale de la partie de stockage (103).

5. Applicateur de la revendication 4, qui comprend un canal (50) permettant une communication fluidique entre un orifice de réception (51) au niveau de la partie de stockage (103) et un orifice de distribution (52) au niveau de la partie de pointe (104) .

6. Applicateur de la revendication 5, dans lequel l'orifice de réception (51) est étanchéifié par le film flexible (20) pendant le stockage de la composition dentaire fluide (30).

7. Applicateur de la revendication 5 ou 6, dans lequel le joint d'étanchéité de l'orifice de réception (51) peut être rompu par l'application d'une pression au compartiment de stockage (201) défini par le film flexible (20) pour ainsi permettre une communication fluidique entre le compartiment de stockage (201) et l'orifice de distribution (52) à travers le canal (50).

8. Applicateur de l'une quelconque des revendications 2 à 7, dans lequel l'élément d'étanchéité (40) comprend au moins une protubérance (41, 42) destinée à mettre en prise et étanchéifier des alésages traversants s'étendant depuis le compartiment de stockage (201) perpendiculairement à l'axe longitudinal (A-A') à travers l'élément allongé rigide (10).

9. Applicateur selon l'une quelconque des revendications précédentes, l'applicateur comprenant une lèvre d'application (60) comprenant des fibres, des poils, un matériau poreux, une mèche, une étoffe, une éponge, un molleton et/ou des filaments.

10. Applicateur selon l'une quelconque des revendications 5 à 8, dans lequel la partie de pointe comprend une aiguille creuse.

11. Applicateur selon l'une quelconque des revendications précédentes, qui comprend un moyen permettant d'appliquer une pression depuis l'extérieur au compartiment de stockage (201) afin d'extruder la composition dentaire fluide (30) à travers le canal (50).

12. Applicateur selon la revendication 11, dans lequel le moyen est fixé de manière pivotante au corps allongé dans une position proximale depuis le compartiment de stockage (201) de sorte que le moyen puisse appliquer une pression au compartiment de stockage (201) défini par le film flexible (20) lorsqu'une pression est exercée sur le moyen.

13. Applicateur selon la revendication 11, dans lequel le moyen est un couvercle protecteur pour le film flexible définissant le compartiment de stockage.

14. Applicateur selon l'une quelconque des revendications précédentes, dans lequel l'orifice de réception (51) permet à la composition dentaire fluide d'entrer dans une première partie de canal (510) essentiellement perpendiculaire à l'axe longitudinal de l'élément allongé (10), et la composition dentaire fluide étant guidée jusqu'à une deuxième partie de canal (520) essentiellement perpendiculaire à la première partie de canal, et la composition dentaire fluide étant guidée jusqu'à l'orifice de distribution (52).

15. Applicateur selon la revendication 14, dans lequel la deuxième partie de canal (520) comprend une partie (521) étanchéifiée par l'élément d'étanchéité.

16. Applicateur selon l'une quelconque des revendications précédentes, qui comprend au moins deux compartiments de stockage.

17. Processus de préparation d'un applicateur selon l'une quelconque des revendications 1 à 16, qui comprend
(a) la fourniture d'un élément allongé rigide (10) ayant une extrémité proximale et une extrémité distale,
(b) la fourniture d'un film flexible (20) sur une surface latérale de l'élément allongé rigide (10) destiné à définir des parois d'un compartiment de stockage (201),
(c) l'introduction d'une composition dentaire fluide (30) dans le compartiment de stockage (201) à travers un alésage traversant, et
(d) l'étanchéification à travers l'alésage traversant avec un élément d'étanchéité (40) pour former un applicateur.

18. Processus de la revendication 17, dans lequel la composition dentaire fluide est introduite dans le compartiment de stockage à travers des alésages s'étendant depuis le compartiment de stockage (201) perpendiculairement à l'axe longitudinal (A-A') de l'élément allongé (10).

19. Processus de la revendication 18, dans lequel au moins deux alésages sont prévus afin de permettre la libération d'air piégé dans le compartiment de stockage à travers un alésage lorsque la composition dentaire est introduite dans le compartiment de stockage à travers un autre alésage.

20. Système d'applicateurs comprenant une pluralité d'applicateurs jetables (1) pour une dose unitaire de composition dentaire fluide (30) tels que définis par l'une quelconque des revendications 1 à 16.

21. Système selon la revendication 20, dans lequel les compositions dentaires fluides sont choisies parmi un adhésif dentaire, un apprêt dentaire, un scellement dentaire, un agent de mordançage dentaire, un vernis dentaire, un composite, une solution de coloration pour tissus durs cariés ou une composition anesthésique locale.
